(19) Europäisches Patentamt
European Patent Office
Office européen des brevets

(11) EP 1 394 215 A1

(12) **EUROPEAN PATENT APPLICATION**
published in accordance with Art. 158(3) EPC

(43) Date of publication:
**03.03.2004 Bulletin 2004/10**

(21) Application number: **02705120.0**

(22) Date of filing: **13.03.2002**

(51) Int Cl.[7]: **C08L 83/04**, A61K 7/00, A61K 7/06, C08G 77/06, A61K 7/075, A61K 7/48, C08G 77/00, C08L 83/00

(86) International application number:
**PCT/JP2002/002346**

(87) International publication number:
**WO 2002/072703 (19.09.2002 Gazette 2002/38)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **13.03.2001 JP 2001071136**

(71) Applicant: **Dow Corning Toray Silicone Co., Ltd.**
**Tokyo 100-0005 (JP)**

(72) Inventors:
- **OZAKI, Masaru,**
**Dow Corning Toray Silicone Co Ltd**
**Ichihara-shi, Chiba 299-0108 (JP)**
- **HAMACHI, Tadashi,**
**Dow Corning Toray Silic. Co Ltd**
**Ichihara-shi, Chiba 299-0108 (JP)**
- **TANAKA, Hidefumi,**
**Dow Corning Toray Silic. Co Ltd**
**Ichihara-shi, Chiba 299-0108 (JP)**

(74) Representative: **Kyle, Diana**
**Elkington & Fife,**
**Prospect House,**
**8 Pembroke Road**
**Sevenoaks, Kent TN13 1XR (GB)**

# (54) POLYORGANOSILOXANE EMULSION, PROCESS FOR PRODUCING THE SAME, AND MATERIAL FOR COSMETIC PREPARATION

(57) A polyorganosiloxane emulsion comprising (A) polyorganosiloxane, (B) N-acylalkyltaurine and/or a salt thereof, and (C) water, the aforementioned emulsion having an average particle diameter of 0.15 µm or more; a method of preparation of the polyorganosiloxane emulsion, characterized by subjecting a polyorganosiloxane (a) having a molecular weight lower than that of component (A), to emulsification polymerization in water in the presence of (B) N-acylalkyltaurine and/or a salt thereof; and a cosmetic raw material comprising the aforementioned polyorganosiloxane emulsion. The cosmetic raw material of the invention possesses excellent cosmetic functionalities such as wettability, smoothness, and good affinity to skin and hair. Furthermore, it is characterized by good stability in storage and compounding stability in combination with our cosmetic raw materials.

EP 1 394 215 A1

## Description

Field of the Invention

The present invention relates to polyorganosiloxane emulsions, methods of preparation thereof, and cosmetic raw materials comprising said polyorganosiloxane emulsions. More specifically, the invention relates to polyorganosiloxane emulsions with excellent storage and compounding stabilities, as well as to cosmetic raw materials with excellent cosmetic functionalities such as affinity to skin, adhesion to hair, good wettability, and smoothness.

Background of the Invention

Polyorganosiloxanes find wide application as raw materials for the preparation of cosmetic products, mold release agents, fiber treatment agents, etc. In particular, in the production of hair and skin cosmetics, they are used as important components that either impart to the cosmetic products such properties as smoothness and wettability required for forming uniform thin films on the surfaces of skin and hair, or produce water-repellant properties and resistance to moisture. When polyorganosiloxanes are used for cosmetic products, they are normally compounded with organic raw materials such as animal and vegetable oils, mineral oils, hydrocarbon oils, fatty acid esters, waxes, alcohols, etc. Therefore it is required that these polyorganosiloxanes should possess good compounding and dispersion stability as well as post-compounding dilution stability with respect to the organic raw materials. In reality, however, the use of polyorganosiloxane for the above purposes encounters some problems because they have low compounding stability with respect to the organic raw materials and reveal tendencies either to thickenining after compounding or to creaming in the course of compounding with separation of the organopolysiloxane components from the compound. Attempts have been made heretofore to improve compounding stability of polyorganosiloxanes with respect to other raw materials by subjecting them, in particular after mixing with surface-active agents and water, to emulsification by applying mechanical energy through the use of colloidal mills, homomixers, homogenizers, combi mixers, or the like. For example, Japanese Patent Publication (Kokoku) No. Sho 58-7335 discloses a silicone emulsion utilizing a cone sugar fatty acid ester, sorbitane fatty acid ester, and a glycerol fatty acid ester as an emulsifying agent. Japanese Patent Application Publication (hereinafter referred to as Kokai) No. Sho 60-126209 discloses a silicone emulsion that utilizes a lipophilic emulsifyer and a polyglycerol fatty acid ester as an emulsifying agent. However, emulsions produced by the methods described above contain large suspended particles of polyorganosiloxane, and the emulsion itself has low storage stability, insufficient dilusion stability after compounding, and low stability against mechanical shear.

On the other hand, known in the art is an emulsification-polymerization silicone emulsion in the form of a microemulsion of a polyorganosiloxane obtained by emulsification polymerization of a low-molecular-weight polyorganosiloxane in the presence of an anionic surface-active agent, catalyst, and water. For example, Kokai No. Hei 9-278626 describes a silicone emulsion utilizing an aliphatic substituted benzenesulfonic acid, aliphatic hydrogensulfite, or a mixture of an unsaturated aliphatic sulfonic acid with a hydrogenated aliphatic sulfonic acid as a surface-active agent. Kokai No. Hei 4-227932(USP 6,316,541) and Kokai No. Hei 9-132646 (EP 755959) also examplify the use of sodium oleylmethyltaurate as an anionic surface-active agent, but the use of dodecylbenzenesulfonic acid is preferable, and practical examples include mainly the dodecylbenzenesulfonic acid. Since the polyorganosiloxane emulsion obtained with the use of the aforementioned surface-active agents contains suspended particles of microscopic sizes, the emulsion shows improved stability in storage and improved stability with respect to dilution and mechanical shear after compounding. However, the surface-active agents used in the composition of this emulsion reveal a degreasing action and have poor affinity to skin as it causes irritation. Furthermore, they result in a low sensitivity improvement effect because of a strong detergent action. For the above reasons, the aforementioned emulsions did not find wide application as cosmetic raw materials.

As a result of study aimed at the solution of the problems of the prior-art technique, the inventors have found that these problems can be solved by utilizing a silicone emulsion emulsified with N-acylalkyltaurine and or/a salt of the latter as a silicone component for cosmetic products. Thus the inventors arrived at the present invention.

It is an object of the present invention to provide a polyorganosiloxane emulsion having excellent storage and compounding stabilities, a method of manufacturing the aforementioned emulsion, and a cosmetic raw material that possesses excellent cosmetic functionalities such as wettability, smoothness and good affinity to skin and hair.

Disclosure of the Invention

The present invention relates to a polyorganosiloxane emulsion having emulsion particles with an average diameter of 0.15 µm or more and composed of (A) polyorganosiloxane, (B) N-acylalkyltaurine and/or a salt of the latter, and (C) water. The invention also relates to a method of preparation of the aforementioned polyorganosiloxane emulsion by subjecting a polyorganosiloxane (a) with the molecular weight lower than that of component (A) to emulsification

polymerization in water in the presence of N-acylalkyltaurine and/or a salt of the latter that constitutes component (B). The invention also relates to a cosmetic raw material comprising the aforementioned polyorganosiloxane emulsion.

Let us first consider a polyorganosiloxane emulsion and method of preparation thereof.

Polyorganosiloxane of component (A) normally has a linear, partially branched, or a branched molecular structure. Silicon-bonded organic groups used in this component comprise substituted or unsubstituted monovalent hydrocarbon groups. Specific examples of such groups are the following: methyl groups, ethyl groups, propyl groups, butyl groups, pentyl groups, hexyl groups, heptyl groups, octyl groups, decyl groups, dodecyl groups, or similar saturated aliphatic hydrocarbon groups; vinyl groups, allyl groups, hexenyl groups or similar unsaturated aliphatic hydrocarbon groups; cyclopentyl groups, cyclohexyl groups, or similar saturated alycyclic hydrocarbon groups; phenyl groups, tolyl groups, naphthyl groups, or similar aromatic hydrocarbon groups, as well as the aforementioned groups in which carbon-bonded hydrogen atoms are partially substituted with halogen atoms or with organic groups comprising epoxy groups, carboxyl group, amino groups, methacryl groups, mercapto groups, etc. Of these, most preferable are methyl groups. The aforementioned polyorganosiloxane may contain a silicon-bonded hydroxyl group and an alkoxy group. The number-average molecular weight of the polyorganosiloxane is preferably in the range of 1000 to 1,000,000, and even more preferably in the range of 5000 to 1,000,000. It is also recommended that at room temperature component (A) be in a liquid state.

Component (B), which is N-acylalkyltaurine and/or a salt thereof, is used as an emulsifying agent for emulsification of component (A) in water. Component (B) is a compound that typically is expressed by the following formula:

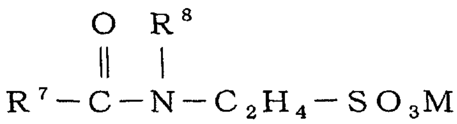

· · · (I),

where $R^7$ and $R^8$ are substituted or unsubstituted monovalent hydrocarbon groups such as methyl groups, ethyl groups, propyl groups, butyl groups, pentyl groups, hexyl groups, heptyl groups, octyl groups, decyl groups, dodecyl groups, myristyl groups, palmityl groups, stearyl groups, or similar saturated aliphatic hydrocarbon groups; vinyl groups, allyl groups, hexenyl groups, oleyl groups, or similar unsaturated aliphatic hydrocarbon groups; cyclopentyl groups, cyclohexyl groups, or similar saturated alycyclic hydrocarbon groups; phenyl groups, tolyl groups, naphthyl groups, or similar aromatic hydrocarbon groups. In the above formula, $R^7$ and $R^8$ may be the same or different, but $R^8$ is normally a methyl group. M may designate a hydrogen atom; sodium, potassium, or another alkali metal; ammonium, triethanolammonium, etc. Component (B) can be exemplified by sodium N-lauroylmethyltaurine, sodium N-myristoylmethyltaurine, sodium N-oleoylmethyltaurine, sodium N-stearoylmethyltaurine, sodium N-coconut oil fatty acid methyltaurate, potassium N-coconut oil fatty acid methyltaurate, magnesium N-coconut oil fatty acid methyltaurate, sodium N-palmytoylmethyltaurate, potassium N-stearoylmethyltaurate, potassium N-cetyloylmethyltaurate, and non-neutralized compounds of the above. These compounds can be used individually or in combinations with each other. It is recommended that component (B) be used in an amount of 1 to 300 parts by weight, preferably 1 to 200 parts by weight, and even more preferably 1 to 100 parts by weight for each 100 parts by weight of component (A). If component (B) is used in an amount less than 1 part by weight or in an amount exceeding 300 parts by weight, the emulsion will become too viscous, will loose flowability, and will become difficult to handle.

Component (C) is water which is used as a medium for emulsification of component (A) by component (B). There are no special restrictions with regard to the amount of component (C) that can be used in the emulsion, provided that after emulsification the emulsion remains stable. It is recommneded, however, to use component (C) in an amount of 10 to 2000 parts by weight for 100 parts by weight of component (A).

The aforementioned polyorganosiloxane emulsion of the invention composed of components (A) thorough (C) can be prepared by subjecting a polyorganosiloxane (a) with the molecular weight lower than that of component (A) to emulsification polymerization in water in the presence of N-acylalkyltaurine (B) and/or salts of the latter. It is recommended that emulsion particles have an average diameter of 0.15 µm or more and not exceeding 100 µm. The polyorganosiloxane of component (a) with the molecular weight lower than that of component (A) can be expressed by the following average unit formula: $R^1_nSiO_{(4-n)/2}$. In this formula, $R^1$ represents substituted or unsubstituted monovalent hydrocarbon groups such as methyl groups, ethyl groups, propyl groups, butyl groups, pentyl groups, hexyl groups, heptyl groups, octyl groups, decyl groups, dodecyl groups, or similar saturated aliphatic hydrocarbon groups; vinyl groups, allyl groups, hexenyl groups or similar unsaturated aliphatic hydrocarbon groups; cyclopentyl groups, cyclohexyl groups, or similar saturated alycyclic hydrocarbon groups; phenyl groups, tolyl groups, naphthyl groups, or similar aromatic hydrocarbon groups, as well as the aforementioned groups in which carbon-bonded hydrogen atoms are partially substituted with halogen atoms or with organic groups comprising epoxy groups, carboxyl group, amino groups, methacryl groups, mercapto groups, etc. Furthermore, a part of $R^1$'s may comprise hydroxyl groups, alkoxy

groups, or hydrogen atoms, but preferably more than 70% of all $R^1$'s comprise methyl groups and more preferably more than 80% of all $R^1$'s comprise methyl groups. In the above formula, n is a number between 0 and 3, preferably between 1.0 and 2.5, and even more preferably between 1.8 and 2.2. The aforementioned polyorganosiloxane of low molecular weight may comprise a cyclic polyorganosiloxane, a linear or branched polyorganosiloxane having molecular terminals capped with triorganosiloxy groups, diorganomonohydroxysiloxy groups, or diorganomonoalkoxysiloxy groups, or mixtures of the above.

The cyclic polyorganosiloxane can be represented by the folowing general formula (II):

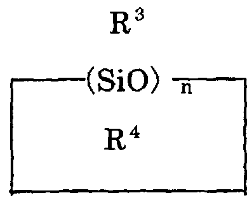

• • • (II) ,

where $R^3$ and $R^4$ are substituted or unsubstituted monovalent hydrocarbon groups that may be the same as groups $R^1$ defined earlier; $R^3$ and $R^4$ may be the same or different, and *n* is an integer from 3 to 8. The following are specific examples of cyclic organopolysiloxanes: hexamethylcyclotrisiloxane, octamethylcyclotetrasiloxane, decamethylcyclopentasiloxane, dodecamethylcyclohexasiloxane, 1,1-diethylhexamethylcyclotetrasiloxane, phenylheptamethylcyclotetrasiloxane, 1,1- diphenylhexamethylcyclotetrasiloxane, 1,3,5,7-tetravinyltetramethylcyclotetrasiloxane, 1,3,5,7-tetramethylcyclotetrasiloxane,1,3,5,7-tetracyclohexyltetramethylcyclotetrasiloxane, tris (3,3,3-trifluoropropyl) trimethylcyclotrisiloxane 1,3,5,7-tetra (3-aminopropyl) tetramethylcyclotetrasiloxane, 1,3,5,7-tetra (N-(2-aminoethyl) 3-aminopropyl) tetramethylcyclotetrasiloxane, 1,3,5,7-tetra (3-mercaptopropyl) tetramethylcyclotetrasiloxane, 1,3,5,7-tetra (3-glycidoxypropyl) tetramethylcyclotetrasiloxane 1,3,5,7-tetra (3-methacryloxypropyl) tetramethylcyclotetrasiloxane, 1,3,5,7-tetra (3-acryloxypropyl) tetramethylcyclotetrasiloxane, 1,3,5,7-tetra (3-carboxypropyl) tetramethylcyclotetrasiloxane, 1,3,5,7-tetra (3-vinyloxypropyl) tetramethylcyclotetrasiloxane, 1,3,5,7-tetra (p-vinylphenyl) tetramethylcyclotetrasiloxane, 1,3,5,7-tetra [3-(p-vinylphenyl) propyl] tetramethylcyclotetrasiloxane, 1,3,5,7-tetra [3-( p-isopropenylbenzoylamino) propyl] tetramethylcyclotetrasiloxane, 1,3,5,7-tetra (N-methacryloyl-N-methyl-3-aminopropyl) tetramethylcyclotetrasiloxane, 1,3,5,7-tetra (N-lauroyl-N-methyl-3-aminopropyl) tetramethylcyclotetrasiloxane, 1,3,5,7-tetra (N-acryloyl-N-methyl-3-aminopropyl) tetramethylcyclotetrasiloxane, 1,3,5,7-tetra (N, N-bis (methacryloyl)-3-aminopropyl) tetramethylcyclotetrasiloxane, and 1,3,5,7-tetra (N,N-bis (lauroyl)-3-aminopropyl) tetramethylcyclotetrasiloxane.

These cyclic organopolysiloxanes can be used individually or in combinations with each other.

Linear polyorganosiloxanes and branched polyorganosiloxanes are siloxanes expressed by the following general formula (III) and (IV):

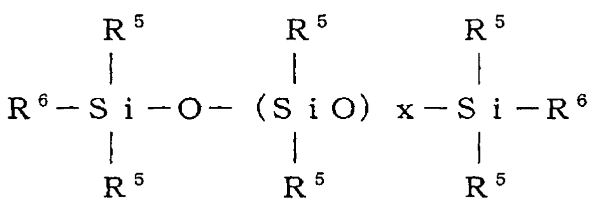

(III)

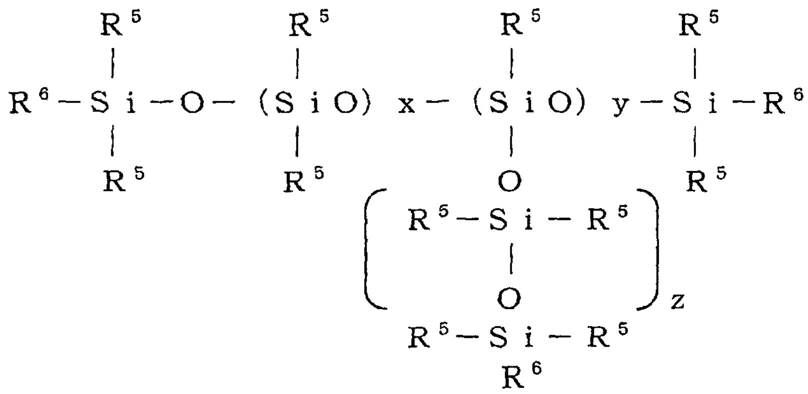 (IV) ,

where R5 and $R^6$ are substituted or unsubstituted monovalent hydrocarbons. Examples of groups $R^5$ and $R^6$ are the same as aforementioned groups $R^1$; $R^5$ and $R^6$ may be the same or different; *x* and *z* are integers from 0 to 100, preferably from 0 to 50, and *y* is an integer from 1 to 100, preferably from 1 to 50. The following are specific examples of the last-mentioned polyorganosiloxanes: α,ω-dihydroxypolydimethylsiloxane, α,ω-dimethoxypolydimethylsiloxane, tetramethyl-1,3-dihydroxydisiloxane, octamethyl-1,7-dihydroxytetrasiloxane, hexamethyl-1,5-diethoxytrisiloxane, hexamethyldisiloxane, and octamethyltrisiloxane.

Component (a) may comprise a cyclic polyorganosiloxane, polydiorganosiloxane having both molecular terminals capped with silanol groups, or a mixture of a cyclic polyorganosiloxane with a linear-chain polydiorganosiloxane.

Emulsification polymerization of the low-molecular-weight polyorganosiloxane (a) can be accompanied by copolymerization with an addition of a hydrolysable organosilane. The aforementioned hydrolysable organosilane can be represented by methyltrimethoxysilane, methyltriethoxysilane, tetraethoxysilane or a similar crosslinking agent, and by hydrolysable organosilanes having organic functional groups. For example, by using a hydrolysable organosilane having an organofunctional group, it becomes possible to introduce an organofunctional group into the polyorganosiloxane of component (A). The following are specific examples of aforementioned hydrolysable organosilanes: 3-aminopropyldiethoxysilane, 3-aminopropyltriethoxysilane, N-(2-aminoethyl)-3-aminopropyltrimethoxysilane, N-(2-aminoethyl)-3-aminopropyldimethoxysilane, 3-chloropropyltrimethoxysilane, 3-chloropropyldimethoxysilane, 3-methacryloxypropyltrimethoxysilane, 3-acryloxypropyldimethoxysilane, 3-glycidoxypropyltrimethoxysilane, 3-glycidoxypropylmethyldimethoxysilane, 3-mercaptopropylmethyldimethoxysilane, 3-carboxypropylmethyldimethoxysilane, p-vinylphenyltrimethoxysilane, 2-(vinylphenyl) ethyltrimethoxysilane, 3-(p-isopropenylbenzoylamino) propyltrimethoxysilane, N-methacryloyl-N-methyl-3-aminopropyltrimethoxysilane, N-lauroyl-N-methyl-3-aminopropylmethyldimethoxysilane, N-acryloyl-N-methyl-3-aminopropyltrimethoxysilane, N-lauroyl-N-methyl-3-aminopropyltrimethoxysilane, N,N-bis (methacryloyl)-3-aminopropylmethyldimethoxysilane, and N,N-bis (lauroyl)-3- aminopropyltrimethoxysilane.

These hydrolysable organosilanes can be used individually or in combinations with each other.

In accordance with the method of the invention, emulsion polymerization may be carried out, e.g., by mixing cyclic polyorganosiloxane (a), N-acylalkyltaurate (B) and water (C), emulsifying component (a) in water, adding an acid catalyst, and conducting emulsification polymerization. The aforementioned acid catalyst contributes to polymerization of the polyorganosiloxane by converting the N-acylalkyltaurate used in emulsification into an acid. The acid catalyst can be represented by a hydrochloric acid, sulfuric acid, phosphoric acid, or acetic acid. The use of such acids as hydrochloric acid or sulfuric acid is preferable in view of their high degree of dissociation. In the case when component (B) is an N-acylalkyltaurine (where $SO_3M$ on the molecular terminal is $SO_3H$), emulsification polymerization can be carried out without addition of the acid catalyst. In emulsification polymerization, after ring-opening polymerization by heating at 50-90 °C cyclic polyorganosiloxane (a) emulsified in water, polymerization may be continued till the desired molecular weight is reached if necessary. Upon completion of polymerization, the N-acylalkyltaurine and the acidic catalyst can be neutralized by adding an alkaline substance. Such a substance may comprise, e.g., sodium hydroxide, potassium hydroxide, ammonia, sodium carbonate, potassium carbonate, ammonium carbonate, potassium acetate, or a similar inorganic substance; or an amine compound such as triethanolamine. If necessary, the magnitude of the molecular weight can be controlled by using a terminal blocking agent in the form of a diorganopolysiloxane having triorganosiloxy groups such as trimethylsiloxy groups. Components (a), (B) and (C) are preferably used in such an amount that 1 to 300 parts by weight of component (B) and 10 to 2000 parts by weight of component (C) are used for each 100 parts by weight of component (a).

The following description relates to the cosmetic raw material of the present invention.

The cosmetic raw material of the present invention comprises the aforementioned organopolysiloxane emulsion. However, for improving compounding stability with cosmetic raw material, within the range not contradicting the objects of the present invention, the emulsion can be combined with some other components known as additives for cosmetic raw materials comprising silicone emulsions. Such additives may comprise anionic surface-active agents other than component (B) and nonionic surface-active agents, pH adjusters, anti-corrosive agents, anti-rust agents, anti-mildew agents, etc. These components can be used individually or in combinations. There are no special restrictions with regard to the sequence in which the components can be added, but it is recommended that components such as non-ionic surface-active agents that delay or hinder emulsification polymerization be added either in a limited amount or after the emulsification polymerization is completed. The following are specific examples of anionic surface-active agents: diethanolamine N-acyl-L-glutamate, triethanolamine N-acyl-L-glutamate, sodium N-acyl-L-glutamate, sodium alkanesulfonate, ammonium alkyl (12, 14, 16) sulfate, triethanolamine (1) alkyl (11, 13, 15) sulfate, triethanolamine (2) alkyl (11, 13, 15) sulfate, triethanolamine (1) alkyl (12, 13, 14) sulfate, triethanolamine alkyl sulfate (liquid), sodium alkyl (12, 13) sulfate, sodium alkyl sulfate (liquid), sodium isoethionate, sodium isostearinlactate, disodium undecylenoyl amidoethyl sulfonsuccinate, triethanolamine olein sulfate, sodium olein sulfate, amidosulfosuccinic disodium oleinate, potassium oleinate, sodium oleinate, morpholine oleinate, oleoylsarcosine, sodium oleoylmethyltaurinate, potassium-containing soap base, potassium-soap base liquid, potash soap, carboxylated polyoxyethylene tridodecyl ether, sodium salt of carboxylated polyoxyethylene tridodecyl ether (3E. O.), triethanolamine N-hardened beef fat fatty acid acyl-L-glutaminate, sodium N-hardened beef fat fatty acid acyl-L-glutaminate, sodium coconut oil fatty acid glycerol sulfate, sodium diundecylenoyl amido ethyl sulfosuccinate, sodium stearylsulfate, potassium stearate, triethanolamine stearate, sodium stearate, sodium N-stearoyl-L-glutamate, disodium stearoyl-L-glutamate, sodium stearoylmethyltaurate, sodium dioctyl sulfosuccinate, sodium dioctyl sulfosuccinate (liquid), disodium sulfosuccinic acid polyoxyethylene monooleylamide (liquid) (2 E.O.), disodium sulfosuccinic acid polyoxyethylene lauroylethanolamide (5 E.O.), disodium sulfosuccinic acid lauryl, diethanolamide cetylsulfate, sodium cetylsulfate, soap base, sodium cetostearylsulfate, triethanolamine tridecyl sulfate, potassium palmitate, sodium palmitate, sodium palmitoyl methyltaurate, sodium castor oil fatty acid (liquid) (30%), ammonium polyoxyethylene alkyl ether sulfate (liquid) (3E. O.), diethanolamine polyoxyethylenealkyl (12, 13) ether sulfate (3E.O.) (liquid), triethanolamine polyoxyethylenealkyl ether sulfate (3E.O.) (liquid), triethanolamine polyoxyethylenealkyl (11, 13, 15) ether sulfate (1E. O.), triethanolamine polyoxyethylenealkyl (12, 13) ether sulfate (3E. O.), sodium polyoxyethylenealkyl ether sulfate (3E. O.) (liquid), sodium polyoxyethylene alkyl (11, 13, 15) ether sulfate (1E. O.), sodium polyoxyethylenealkyl (11 to 15) ether sulfate (3E. O.), sodium polyoxyethylenealkyl (12, 13) ether sulfate (3E. O.), sodium polyoxyethylenealkyl (12 to 14) ether sulfate (3E. O.), sodium polyoxyethylenealkyl (12 to 15) ether sulfate (3E. O.), disodium polyoxyethylenealkyl (12 to 14) sulfosuccinate (7E. O.), sodium polyoxyethylene undecyl ether sulfate, sodium polyoxyethyleneoctylphenyl ether sulfate (liquid), ammonium polyoxyethyleneoleyl ether sulfate, lauryldisodium polyoxyethylene sulfosuccinate, sodium polyoxyethylene nonylphenyl ether sulfate, sodium polyoxyethylene pentadecyl ether sulfate, triethanolamine polyoxyethylene myristyl ether sulfate, sodium polyoxyethylene myristyl ether sulfate (3E. O.), sodium polyoxyethylene lauryl ether acetate (16 E.O.) (liquid), ammonium polyoxyethylene lauryl ether sulfate (2E. O.), triethanolamine polyoxyethylene lauryl ether sulfate, sodium polyoxyethylene lauryl ether sulfate, diethanolamine myristyl sulfate, sodium myristic sulfate, potassium myristic sulfate, sodium N-myristoyl-L-glutamate, sodium myristoylmethylamino acetate, sodium myristoylniethyl-β-alanine (liquid), sodium myristoyl methyltaurate, medicinal soap, triethanolamine/magnesium coconut oil alkyl sulfate, triethanolamine N-coconut oil fatty acid acyl-L-glutamate, sodium N-coconut oil fatty acid acyl-L-glutamate, sodium N-coconut oil fatty acid ethylester sulfonate, potassium coconut oil fatty acid, potassium coconut oil fatty acid (liquid), N-coconut oil fatty acid - sodium hardened beef fat fatty acid acyl-L-glutamate, coconut oil fatty acid sarcosine, triethanolamine coconut fatty acid sarcosine, sodium coconut oil fatty acid sarcosine, triethanolamine coconut oil fatty acid, triethanolamine coconut oil fatty acid (liquid), sodium coconut fatty acid, sodium coconut fatty acid methyl taurate, sodium coconut fatty acid methyl alanine (liquid), potassium coconut oil fatty acid methyl alanine, sodium coconut oil fatty acid methyl taurate, sodium laurylaminodipropionate, sodium laurylaminodipropionate, (liquid) (30%), sodium laurylsulfoacetate, sodium laurylbenzenesulfonate, laurylsulfuric acid, ammonium laurylsulfate, potassium laurylsulfate, diethanolamine laurylsulfate, triethanolamine laurylsulfate, sodium laurylsulfate, magnesium laurylsulfate, monoethanolamine laurylsulfate, potassium laurate, triethanolamine laurate, triethanolamine laurate (liquid), sodium laurate, triethanolamine lauric acid myristate, triethanolamine lauroyl-L-glutamate, sodium N-lauroyl-L-glutamate, lauryl sarcosine, potassium lauryl sarcosine, lauryl sarcosine triethanolamine (liquid), sodium lauryl sarcosine, sodium laurylmethyl-β-alanine (liquid), sodium lauroylmethyltaurate, and sodium lauroylmethyltaurate (liquid).

The following are examples of nonionic surface-active agents: ethylene glycol fatty acid ethyls, polyethylene glycol fatty acid esters, propyleneglycol fatty acid esters, polypropyleneglycol fatty acid esters, glycol fatty acid esters, trimethylolpropane fatty acid esters, pentaerythritol fatty acid esters, glycoside derivatives, glycerol alkylether fatty acid esters, fatty acid amides, alkylolamides, alkylamineoxides, lanolin and derivatives thereof, castor oil derivatives, hardened castor oil derivatives, styrol and derivatives thereof, polyoxyethylenealkyl ether, polyoxyethylenealkylallyl ether, polyoxyethylenealkylamines, polyoxyethylene fatty acid amide, polyoxyethylene alkylolamide, polyoxyethylene diethanolamine fatty acid ester, polyoxyethylenetrimethylolpropane fatty acid ester, polyoxyethylenealkylether fatty acid

ester, polyoxyethylene polyoxypropyleneglycol, polyoxyethylene- polyoxypropylenealkylether, polyoxyethylenepolyoxypropylene polyhydric alcohol, glycerol fatty acid ester, polyglycerol fatty acid ester, polyoxyethyleneglycerol fatty acid ester, sorbitane fatty acid ester, polyoxyethylene sorbitane fatty acid ester, and cane sugar fatty acid ester.

pH adjusters can be represented by a hydrochloric acid, sulfuric acid, phosphoric acid, diammonium hydrogenphosphate, disodium hydrogenphosphate, dipotassium hydrogenphosphate, ammonium dihydrogenphosphate, sodium dihydrogenphosphate, potassium dihydrogenphosphate, sodium triphosphate, potassium triphosphate, acetic acid, ammonium acetate, sodium acetate, potassium acetate, citric acid, sodium citrate, diammonium citrate, sodium carbonate, potassium carbonate, ammonium carbonate, sodium hydrogencarbonate, ammonium hydrogencarbonate, sodium hydroxide, potassium hydroxide, ammonia, and triethanolamine.

Given below are examples of compounds that can be used as anti-corrosive agents, anti-rust agents, and anti-mildew agents: benzoic acid, aluminum benzoate, sodium benzoate, isopropylmethylphenol, ethylhexanediol, chlorinated lysozyme, chlorohexyzinehydrochloride, octylphenoxyethanol, orthophenylphenol, sodium perborate, Photosensitizer No. 101, Photosensitizer No. 201, Photosensitizer No. 301, Photosensitizer No. 401, chlorohexyzine gluconate (liquid), cresol, Chloramine T, chloroxylenol, chlorocrezol, chlorophoenicin, chlorohexyzine, chlorobutanol, resorcin acetate, salicylic acid, sodium salicylate, domisphene bromide, zinc pyrithione, zinc pyrithione (liquid), sorbic acid, potassium sorbate, thiantol, thioxolone, thymol, thiram, dehydroacetic acid, sodium dehydroacetate, trichlorocarbanilide, trichlorohydroxydiphenyl ether, isobutyl paraoxybenzoate, isopropyl paraoxybenzoate, ethyl paraoxybenzoate, butyl paraoxybenzoate, propyl paraoxybenzoate, benzyl paraoxybenzoate, methyl paraoxybenzoate, sodium methylparaoxybenzoate, parachlorophenol, sodium paraphenolsulfonate (dihydrate), halocarbane, phenoxyethanol, phenol, hexachlorophane, mononitroguaiacol, sodium mononitroguaiacol, iodine paradimethyl aminostyrylheptylmethyl liazolinium, lauryl trimethylammonium trichlorophenoxide, oxyquinoline sulfate, oxyquinoline phosphate, and resorcin.

Skin cosmetics having excellent affinity to skin, smoothness and wettability can be obtained by adding the cosmetic raw materials of the invention to various other raw materials which are listed below, and mixing them. Apart from the aforementioned anionic surface-active agents, nonionic surface-active agents, pH adjusters, anti-corrosive agents, anti-rust agents, and anti-mildew agents, these raw material additives for skin cosmetics can be exemplified by avocado oil, almond oil, olive oil, cacao butter, sesami oil, wheat germ oil, safflower oil, shea butter, turtle oil, camellia oil, persic oil, castor oil, grape oil, macadamia nut oil, mink oil, egg yolk oil, Japan wax, coconut oil, rose hip oil, hydrogenated oil, or similar oils or fats; orange roughy oil, carnauba wax, candelilla wax, whale tallow, jojoba oil, montan wax, beeswax, lanolin, or similar waxes; liquid paraffin, Vaseline, paraffin, ceresin, microcrystalline wax, squalan, or similar hydrocarbons; lauric acid, myristic acid, palmitic acid, stearic acid, oleic acid, behenic acid, undecylenic acid, oxystearic acid, linilic acid, lanoleic acid, synthetic fatty acids such as higher fatty acids; ethyl alcohol, isopropyl alcohol, lauryl alcohol, cetyl alcohol, cetostearyl alcohol, stearyl lacohol, oleyl alcohol, behenyl alcohol, lanolin alcohol, hydrogenised lanolin alcohol, hexyldecanol, octyldodecanol, isostearyl alcohol, or similar alcohols; cholesterol, dihydrocholesterol, phytosterol, or similar sterols; ether lenoleate, isopropyl myristate, lanolin fatty acid isopropyl, hexyl lanoleate, myristyl myristate, cetyl myristate, octyldodecyl myristate, decyl oleate, octyldodecyl oleate, hexyldecyl dimethyloctanate, cetyl isooctanate, cetyl palmitate, glycerol trimyristate, tri (capryl-caprylic acid) glycerol, propyleneglycol dioleate, glycerol triisostearate, glycerol triisooctanate, cetyl lactate, myristyl lactate, diisostearyl maleate, or similar fatty acid esters; glycerin, propylene glycol, 1,3-butylene glycol, polyethylene glycol, sodium d, 1-pyrrolidone carboxylate, sodium lactate, sorbitol, sodium hyaluronate, or similar moistening agents; cationic surface-active agents; betainic-type, amino-acid-type, imidasolic-type, and lecitinic-type amphoteric surface-active agents; pigments such as iron oxide or similar coloring pigments, zinc oxide, titanium oxide, zirconium oxide, or similar white pigments, mica, talk, sericite, or similar filler extenders; dimethylpolysiloxane, methylphenylpolysiloxane, octamethytetracyclosiloxane, decamethylcyclopentasiloxane, polyether-modified silicone oil, amino-modified silicone oil, or similar silicone oils; purified water; carrageenan, alginic acid, Arabia gum, traganth, pectin, starch, xanthan gum, polyvinyl alcohol, polyvinyl pyrrolidone, sodium polyacrylate, polyethylene glycol, or similar thickeners; silicone-acryl copolymer, silicone resin, acryl polymer, or similar film-forming agents; as well as ultraviolet-ray absorbers, disinfectants, anti-inflammatory agents, antiperspiration agents, aromodorants, antioxidants, and propellants. Examples of products that may use the aforementioned skin cosmetic materials are hand creams, skin creams, foundation creams, eye shadows, cleansing creams, and body shampoos.

When the cosmetic raw materials of the present invention are used for preparation of hair cosmetic materials, in addition to the aforementioned anionic surface-active agents, non-ionic surface-active agents, pH adjusters, anti-corrosive agents, anti-mildew agents, etc., the composition may also be compounded with film-forming agents, freeze-prevention agents, oily components, emulsifiers, wetting agents, anti-dandruff agents, antioxidants, chelate agents, ultraviolet-ray absorbers, odorants, dyes, or other additives that may improve adherence to hair or to impart to the obtained hair cosmetics excellent smoothness and wettability. The following are specific examples of film-forming agents: polymers of (meth)acrylic radical-polymerizable monomers, copolymers of silicone-type compounds and (meth)acrylic radical-polymerizable monomers, poly (N-acylalkyleneimine), poly (N-methylpyrrolidone), silicone resins

modifies with fluoro-containing organic groups or amino groups, and non-functional silicone resins. There are no special restrictions with regard to freeze-protection agents, but in general these agents can be represented by such substances as ethanol, isopropyl alcohol, 1,3-butylene glycol, ethylene glycol, propylene glycol, and glycerol. Oily components may be those normally used for the preparation of conventional cosmetics. Typical examples of these substances are microcrystalline wax, paraffin wax, spermaceti, bees wax, Japan wax, sugar-cane wax, or similar waxes or their mixtures, liquid paraffin, α-olefin oligomer, squalan, squalene, or similar hydrocarbon oils or their mixtures, cetanol, stearyl alcohol, isostearyl alcohol, hardened castor oil derivative alcohol, behenyl alcohol, lanolin alcohol, or similar linear-chain or branched-chain, saturated or unsaturated, unsubstituted or hydroxy-substituted higher alcohols or their mixtures, palmitic acid, myristic acid, oleic acid, stearic acid, hydroxystearic acid, isostearic acid, behenic acid, castor oil fatty acid, coconut oil fatty acid, beef-fat fatty acid, or similar linear-chain or branched-chain, saturated or unsaturated, unsubstituted or hydroxy-substituted higher fatty acids or their mixtures, olive oil, coconut oil, rapeseed oil, palm oil, castor oil, hardened castor oil, peanut oil, beef fat, hydrogenated beef fat, jojoba oil, hardened jojoba oil, glycerol monostearate, monooleic acid glyceride, glycerol dipalmitate, glycerol trimyristate, oleyl oleate, isostearyl isostearate, palmityl behenate, isopropyl palmitate, stearyl acetate, ester dihydroxystearate, or similar esters, linear-chain, branched-chain, or cyclic low-molecular-weight silicone oils, amino-modified silicone oils, fatty-acid-modified silicone oils, alcohol-modified silicone oils, polyether-modified silicone oils, phosphoric-acid (basic) group containing silicone oil, sulfuric acid (basic) group containing silicone oil, fluoro-modified alkyl-group containing silicone oil, alkyl-modified silicone oil, epoxy-modified silicone oil, or similar silicone oils, high-molecular-weight silicone, and silicone resins that are soluble in solvents, liquid or rubbery at room temperature, or possess thermoplastic properties, or mixture of the aforementioned silicone oil and silicone resins. It is recommended to use the aforementioned silicone in the form of emulsions, and the emulsifiers may comprise, e.g., glycerol monostearate, sorbitane monopalmitate, polyoxyethylene cetyl ether, polyoxyethylene stearic acid ether, polyoxyethylene sorbitane monolaurate, or other emulsifiers normally used for these purposes. Wetting agents may be represented by hexylene glycol, polyethylene glycol 600, sodium pyroglutamate, and glycerol. Antidandruff agents may be represented by sulfur, selenium sulfate, zinc pyridium-1-thiol-N-oxide, salicylic acid, 2,4,4'-trichloro-2'-hydroxydiphenyl ether, and 1-hydroxy-2- pyridine compound. Antioxidants may comprise BHA, BHT, and γ-oryzanol. Chelate agents may comprise ethyl diamine tetraacetic acid, citric acid, ethane-1-hydroxy-1,1-diphosphonic acid, or salts of the aforementioned acids. Ultraviolet-ray-absorbants can be represented by benzophenone derivatives such as 2-hydroxy-4-methoxybenzophenone, benzotriazol derivatives such as 2-(2'-hydroxy-5'-methyl-phenyl)-benzotriazol, and cinnamonic acid ester. Furthermore, if necessary, the cosmetic material of the invention can also be compounded with various other components, such as glycerin, propylene glycol, dipropylene glycol, 1,3-butylene glycol, or similar polyhydric alcohols, salts of monoalkyltrimethyl ammonium, salts of dialkyldimethyl ammonium, preferably quaternary ammonium salts such as stearyltrimethyl ammonium chloride, behenyltrimethyl ammonium chloride, distearyldimethyl ammonium chloride, dibehenyldimethyl ammonium chloride, or similar cationic surface-active agents or amphoteric surface-active agents, squalene, lanolin, perfluoropolyether, cationic polymers or similar sensitization improvers, propylene glycol, glycerin, sorbitol, or similar moistening agents, methyl cellulose, carboxyvinyl polymer, hydroxyethyl cellulose, polyoxyethylene glycol distearate, ethanol or similar viscosity adjusters, pearl-like-hue-imparting agents, odorants, dyes, pigments, propellants, vitamins, hair nutrients, hormones, or similar pharmaceutical substances, triclosan, triclocarban, or similar disinfectants, potassium glycyrrhizinate, tocopherol acetate, or similar anti-inflammatory agents, zinc pyrithione and octopirox, or similar anti-dandruff agents, methylparaben and butylparaben, or similar antiseptics, propellants, and other components described in Encyclopedia of Shampoo Ingredients (Miscelle Press, 1985). The following are examples of hair-cosmetic products or which the cosmetic raw material of the invention is applicable: shampoos, hair rinses, hair conditioners, hair treatments, setting lotions, blow-styling lotions, blow-styling agents, hair sprays, styling foams, styling jels, hair liquids, hair tonics, hair creams, hair nutrients, hair-growth stimulators, and hair dye compositions.

Description of Preferred Embodiments of the Invention

The invention will now be described in more detail with reference to practical examples. In these examples, all "parts" are "parts by weight", and all "%" are "wt.%". Physical properties and stability of the polyorganosiloxane emulsions were evaluated by the methods described below.

Average Diameter of Emulsion Particles

This parameter was measured with the use of a Coulter Model No. 4 measurement instrument (the product of Coulter Electronics).

Number-Average Molecular Weight of Polyorganosiloxane

The polyorganosiloxane emulsion was decomposed by adding an alcohol, the polyorganosiloxane component was dissolved in a solvent, and the number-average molecular weight of the polyorganosiloxane was determined with the use of a gas-permeation chromatography analyzer (Model S-8120, Shimazu Seisakusho Co., Ltd.). The measured value was recalculated with the use of polystyrene as a standard.

Non-Volatile Components

About 2 g of the polyorganosiloxane emulsion were placed onto a flat-bottom plate and the weight was determined with precision scales. The weight was determined for the second time after the plate was heated for 2 hours at 105°C in a hot-air-circulation oven for evaporation of volatile components. The weight of non-volatile components was determined as a weight difference between the weight of the plate with the emulsion prior and after the heating. Percent content of non-volatile components was determined by means of the following formula:

Non-volatile Content (%) = [(Weight of Emulsion) - (Weight of Volatile Components)]/ (Weight of Emulsion)

Emulsion Stability

The obtained polyorganosiloxane emulsion itself and a water-diluted solution containing 10% the obtained polyorganosiloxane emulsion were poured into 100 $cm^3$ glass bottles, and the bottles were tightly closed. The bottles were retained for 30 days in a circulation-environment test chamber set to a temperature cycle with temperatures from 0 to 50°C variable for every 12 hours. After the 30-day period, the emulsions were observed with regard to their appearance and oil components on the surface. The following criteria were used for evaluation:

<Change in Appearance>

◎ Uniform, and any change is not observed.
○ Some creaming is observed in the upper part.
Δ Creaming is noticeable.
X Separation into two layers.

<Oil on the Surface>

○ Oil is absent
Δ Some traces of oil are seen.
X Oil on the entire surface.

[Practical Example 1]

A uniform mixture was prepared by adding 399.9 parts of octamethylcyclotetrasiloxane and 0.1 parts of hexamethyldisiloxane to a liquid mixture of 20 parts of sodium N-lauroylmethyltaurinate and 450 parts of ion-exchange water. After the components were uniformly mixed, the mixture was treated twice at a pressure of 40 MPa in a high-pressure homogenizer, whereby a uniform emulsion was obtained. The emulsion was then transferred to a separable flask equipped with a condenser, nitrogen-supply port, and a stirrer. After 60 parts of an aqueous solution of 3.5% hydrochloric acid were added to the emulsion while the latter was stirred, the temperature of the emulsion was raised to 85°C, the emulsion was retained at that temperature for 6 hours, and held at 40°C for another 8 hours, and as a result was subjected to emulsification polymerization. Upon completion of the emulsification polymerization, an aqueous solution of 5% sodium hydroxide was added in an amount required for adjusting emulsion pH to 7. As a result, a polydimethylsiloxane emulsion was prepared. This emulsion was designated A-1 and was subjected to measurement of physical properties and stability. The results are shown in Table 1.

[Practical Example 2]

A uniform mixture was prepared by adding 400 parts of octamethylcyclotetrasiloxane to a liquid mixture of 20 parts of sodium N-lauroylmethyltaurinate and 450 g of ion-exchange water. The mixture was treated twice at a pressure of 40 MPa in a high-pressure homogenizer, whereby a uniform emulsion was obtained. The emulsion was then transferred to a separable flask equipped with a condenser, nitrogen-supply port, and a stirrer. After 100 parts of an aqueous solution of 3.5% hydrochloric acid were added to the emulsion while the latter was stirred, the temperature of the emulsion was raised to 85°C, the emulsion was retained at that temperature for 4 hours, and held at 20°C for another 5 hours, and as a result was subjected to emulsification polymerization. Upon completion of the emulsification polymerization, an aqueous solution of 5% sodium hydroxide was added in an amount required for adjusting emulsion pH to 7. As a result, a polydimethylsiloxane emulsion was prepared. This emulsion was designated A-2 and was subjected to measurement of physical properties and stability. The results are shown in Table 1.

[Practical Example 3]

A uniform mixture was prepared by adding 400 parts of octamethylcyclotetrasiloxane to a liquid mixture of 20 parts of sodium N-lauroylmethyltaurinate and 450 g of ion-exchange water. The mixture was treated twice at a pressure of 40 MPa in a high-pressure homogenizer, whereby a uniform emulsion was obtained. The emulsion was then transferred to a separable flask equipped with a condenser, nitrogen-supply port, and a stirrer. After 100 parts of an aqueous solution of 3.5% hydrochloric acid were added to the emulsion while the latter was stirred, the temperature of the emulsion was raised to 85°C, the emulsion was retained at that temperature for 4 hours, and held at 5°C for another 14 hours, and as a result was subjected to emulsification polymerization. Upon completion of the emulsification polymerization, an aqueous solution of 5% sodium hydroxide was added in an amount required for adjusting emulsion pH to 7. As a result, a polydimethylsiloxane emulsion was prepared. This emulsion was designated A-3 and was subjected to measurement of physical properties and stability. The results are shown in Table 1.

[Practical Example 4]

A uniform mixture was prepared by adding 395 parts of decamethylcyclopentasiloxane to a liquid mixture of 25 parts of sodium N-lauroylmethyltaurinate and 450 g of ion-exchange water. The mixture was treated twice at a pressure of 40 MPa in a high-pressure homogenizer, whereby a uniform emulsion was obtained. The emulsion was then transferred to a separable flask equipped with a condenser, nitrogen-supply port, and a stirrer. After 100 parts of an aqueous solution of 3.5% hydrochloric acid were added to the emulsion while the latter was stirred, the temperature of the emulsion was raised to 85°C, the emulsion was retained at that temperature for 4 hours, and held at 25°C for another 3 hours. The content was then combined with 5 parts γ-glycidoxypropyltrimethoxysilane that were slowly added, the mixture was held for 2 hours at 25°C and as a result was subjected to emulsification polymerization. Upon completion of the emulsification polymerization, an aqueous solution of 5% sodium hydroxide was added in an amount required for adjusting emulsion pH to 7. As a result, a polydimethylsiloxane emulsion was prepared. This emulsion was designated A-4 and was subjected to measurement of physical properties and stability. The results are shown in Table 1.

[Comparative Example 1]

A liquid mixture was prepared from 400 parts of a 74,400 molecular-weight dimethylpolysiloxane capped with trimethylsiloxy groups, 20 parts of a polyoxyethylene (4) lauryl ether, 20 parts of a polyoxyethylene (20) cetyl ether, and 40 parts of ion-exchange water. After emulsification of the obtained mixture in a vacuum-type emulsifier (the product of Tokushu Kika Kogyo Co., Ltd.) , the product was combined with 520 parts of ion-exchange water, and a silicone emulsion was obtained. This emulsion was designated B-1 and was subjected to measurement of physical properties and stability. The results are shown in Table 2.

[Comparative Example 2]

After addition of 400 parts of octamethylcyclotetrasiloxane to a liquid mixture of 450 parts of ion-exchange water and 20 parts of an anionic surface-active agent, in turn comprising a mixture of 75% of sodium tetradecenesulfonate and 25% of sodium hydroxytetradecanesulfonate, the components were uniformly mixed, and the obtained mixture was passed 2 times through a high-pressure homogenizer operating under pressure of 40 MPa. As a result, a uniform emulsion was produced. The emulsion was then transferred to a separable flask equipped with a condenser, nitrogen-supply port, and a stirrer. After 100 parts of an aqueous solution of 3.5% hydrochloric acid were added to the

emulsion while the latter was stirred, the temperature of the emulsion was raised to 85°C, the emulsion was retained at that temperature for 3 hours, and held at 5°C for another 14 hours. As a result the emulsion was subjected to emulsification polymerization. Upon completion of the emulsification polymerization, an aqueous solution of 5% sodium hydroxide was added in an amount required for adjusting emulsion pH to 7. As a result, a silicone emulsion was prepared. This emulsion was designated B-2 and was subjected to measurement of physical properties and stability. The results are shown in Table 2.

[Comparative Example 3]

After addition of 395 parts of decamethylcyclopentasiloxane to a liquid mixture of 450 parts of ion-exchange water and 25 parts of dodecylbenzenesulfonic acid, the components were uniformly mixed, and the obtained mixture was passed 2 times through a high-pressure homogenizer operating under pressure of 40 MPa. As a result, a uniform emulsion was produced. The emulsion was then transferred to a separable flask equipped with a condenser, nitrogen-supply port, and a stirrer. After 100 parts of an aqueous solution of 3.5% hydrochloric acid were added to the emulsion while the latter was stirred, the temperature of the emulsion was raised to 85°C, the emulsion was retained at that temperature for 4 hours, and then held at 25°C for another 3 hours. The emulsion was then combined with 5 parts of γ-glycidoxypropyltrimethoxysilane which was slowly added, and then was held for another 2 hours at 25°C. As a result the emulsion was subjected to emulsification polymerization. Upon completion of the emulsification polymerization, an aqueous solution of 5% sodium carbonate was added in an amount required for adjusting emulsion pH to 7. As a result, a silicone emulsion was prepared. This emulsion was designated B-3 and was subjected to measurement of physical properties and stability. The results are shown in Table 2.

[Table 1]

| Sample No. / Characteristics | Practical Examples | | | |
|---|---|---|---|---|
| | A-1 | A-2 | A-3 | A-4 |
| Appearance | Milky white uniform liquid | Milky white uniform liquid | Milky white uniform liquid | Milky white uniform liquid |
| Average particle size (μm) | 0.18 | 0.18 | 0.18 | 0.18 |
| Non-volatile components (%) | 39.2 | 39.0 | 38.9 | 39.2 |
| Molecular weight of polysiloxane | 73,600 | 102,000 | 148,000 | 152,000 |
| Stability of emulsion itself | | | | |
| Change in appearance | ◎ | ◎ | ◎ | ◎ |
| Oil on the surface | ○ | ○ | ○ | ○ |
| Stability of water-diluted solution | | | | |
| Change in appearance | ◎ | ◎ | ◎ | ◎ |
| Oil on the surface | ○ | ○ | ○ | ○ |

[Table 2]

| Sample No. / Characteristics | Comparative Examples | | |
|---|---|---|---|
| | B-1 | B-2 | B-3 |
| Appearance | Milky white uniform liquid | Milky white uniform liquid | Milky white uniform liquid |
| Average particle size (μm) | 0.18 | 0.18 | 0.18 |
| Non-volatile components (%) | 44.0 | 39.2 | 39.0 |
| Molecular weight of polysiloxane | 74,400 | 150,000 | 151,000 |
| Stability of emulsion itself | | | |
| Change in appearance | Δ | ◎ | ◎ |
| Oil on the surface | X | Δ | Δ |
| Stability of water-diluted solution | | | |
| Change in appearance | Δ | ◎ | ◎ |
| Oil on the surface | X | Δ | Δ |

water-diluted solution

[Practical Example 5]

Hair shampoo compositions A-5 to A-7 with contents shown in Table 3 were prepared with the use-of samples A-1 to A-3 produced in Practical Examples 1 to 3. These shampoo compositions were subjected to measurement of physical properties and stability. Evaluation was carried out by the methods described below. The results are shown in Table 3.

<Stability>

Stability was evaluated by observing the shampoo directly after the preparation and after 1-month storage at 50°C. The following criteria were used for evaluation:

◎ Uniform, no changes.

○ Some creaming is observed in the upper part.

Δ Creaming is noticeable.

X Separation into two layers.

Hair Touch Sensation

<Hair Pretreatment>

5 g lock of 20 cm-long hair was washed in an a aqueous solution of a 2.5 wt.% sodium polyoxyethylenelaurate ether sulfate, rinsed in a flow of water, and then dried for more than 12 hours at 25°C.

<Treatment with Shampoo>

The hair pretreated as described above was treated with 1 g of the shampoo composition and rinsed for 1 min. in a flow of water. The water was then drained. After brushing until no resistance to brushing is sensed, the hair was dried for 12 hours at 25°C. After repeating the above procedure 5 times, hair touch sensation was evaluated by a panel of 5 people.

Sensation of Hair Smoothness

<Hair Pretreatment>

5 g lock of 20 cm-long hair was washed in an a aqueous solution of a 2.5 wt.% sodium polyoxyethylenelaurate ether sulfate, rinsed in a flow of water, and then dried for more than 12 hours at 25°C.

<Treatment with Shampoo>

The hair pretreated as described above was treated with 1 g of the shampoo composition and rinsed for 1 min. in a flow of water. The water was then drained. After brushing until no resistance to brushing is sensed, the hair was dried for 12 hours at 25°C. The above procedure was repeated 5 times.

<Evaluation of Sensation of Smoothness>

A lock of hair treated with the shampoo by the method described above was placed onto the sample table of a friction sense tester (*KES-SE* Model, the product of Kato Tech Ltd.) and fixed at both ends. After the friction tip was set to a load of 25 g, it was caused to slide over the sample with the speed of 0.5 mm/sec, and then MIU (mean coefficient of friction) and MMD (variation in the mean coefficient of friction) were measured. The following formulae were used for calculating a coefficient of improvement in slipperiness and a coefficient of improvement in smoothness:

$$\text{Coefficient of improvement in slipperiness (\%)} = [(A-B)/A] \times 100,$$

where: A is MIU of hair treated with the shampoo composition without the addition of silicone; and B is MIU of hair treated with the shampoo composition with the addition of silicone.

$$\text{Coefficient of improvement in smoothness (\%)} = [(C-D)/C] \times 100,$$

where: C is MMD of hair treated with the shampoo composition without the addition of silicone; and D is MMD of hair treated with the shampoo composition with the addition of silicone.

[Comparative Example 5]

For comparison, hair shampoo compositions B-4 and B-5 prepared with the use of samples B-1 and B-2 produced in Comparative Examples 1 and 2 were evaluated for stability and with regard to characteristics by the same method as in Practical Example 5. The results are shown in Table 3.

[Table 3]

| Shampoo Composition No. | | Practical Examples | | | Comp. Examples | |
|---|---|---|---|---|---|---|
| | | A-5 | A-6 | A-7 | B-4 | B-5 |
| COMPOSITION (%) | Sodium N-lauroylmethyl-taurinate (25% aqueous solution) | 25.0 | 25.0 | 25.0 | 25.0 | 25.0 |
| | Lauroyl sarcosine sodium | 6.0 | 6.0 | 6.0 | 6.0 | 6.0 |
| | Lauroyl dimethyl betaine (25% aqueous solution) | 10.0 | 10.0 | 10.0 | 10.0 | 10.0 |
| | Coconut oil fatty acid diethanolamide | 4.0 | 4.0 | 4.0 | 4.0 | 4.0 |
| | Propylene glycol | 5.0 | 5.0 | 5.0 | 5.0 | 5.0 |
| | Phenoxyethanol | 1.0 | 1.0 | 1.0 | 1.0 | 1.0 |
| | Chlorinated 0-[2-hydroxy-3-(trimethylammonia) propyl] hydroxyethyl cellulose | 0.5 | 0.5 | 0.5 | 0.5 | 0.5 |
| | Sample A-1 | 10.0 | - | - | - | - |
| | Sample A-2 | - | 10.0 | - | - | - |
| | Sample A-3 | - | - | 10.0 | - | - |
| | Sample B-1 | - | - | - | 10.0 | - |
| | Sample B-2 | - | - | - | - | 10.0 |
| | Ion-exchange water | 38.5 | 38.5 | 38.5 | 38.5 | 38.5 |
| Stability | Directly after preparation | ◎ | ◎ | ◎ | ○ | ◎ |
| | After 1 month at 50°C | ◎ | ◎ | ◎ | Δ | ○ |

[Practical Example 6]

500 parts of a α, ω-dihydroxypolydimethylsiloxane with a molecular weight of 3000 (polymerization degree 40) were added to a liquid mixture of 10 parts of sodium N-lauroylmethyltaurinate and 390 parts of ion-exchange water, the components were uniformly mixed, an the mixture was twice passed through a high-pressure homogenizer operating under a pressure of 40 MPa. As a result, a uniform emulsion was obtained. The emulsion was then transferred to a separable flask equipped with a condenser, nitrogen-supply port, and a stirrer. After 50 parts of an aqueous solution of 7% hydrochloric acid were added to the emulsion while the latter was stirred, emulsification polymerization was carried out by retaining the emulsion for 24 hours at 25°C. Upon completion of the emulsification polymerization, an aqueous solution of 5% sodium carbonate was added in an amount required for adjusting emulsion pH to 7. As a result, a polydimethylsiloxane emulsion was prepared. This emulsion had an average particle size of 0.48 μm, and the number-average molecular weight of polydimethylsiloxane was 148,000. This emulsion was designated as Sample A-8.

[Practical Example 7]

499 parts of a α, ω-dihydroxypolydimethylsiloxane with a molecular weight of 3000 (polymerization degree 40) were added to a liquid mixture of 10 parts of sodium N-lauroylmethyltaurinate and 390 parts of ion-exchange water, the components were uniformly mixed, an the mixture was twice passed through a high-pressure homogenizer operating under a pressure of 40 MPa. As a result, a uniform emulsion was obtained. The emulsion was then transferred to a separable flask equipped with a nitrogen-supply port and a stirrer. After 50 parts of an aqueous solution of 7% hydrochloric acid were added to the emulsion while the latter was stirred, the emulsion was retained for 12 hours at 25°C, combined with 1 part of a slowly added methyltriethoxysilane, and held for 12 hours at 25°C for emulsification polymerization. Upon completion of the emulsification polymerization, an aqueous solution of 5% sodium carbonate was added in an amount required for adjusting emulsion pH to 7. As a result, a polyorganosiloxane emulsion was prepared. This emulsion had an average particle size of 0.52 μm, and the number-average molecular weight of polydimethylsiloxane was 152,000. This emulsion was designated as Sample A-9.

[Comparative Example 6]

500 parts of a α, ω-dihydroxypolydimethylsiloxane with a molecular weight of 3000 (polymerization degree 40) were added to a liquid mixture of 390 parts of ion-exchange water with 10 parts of an anionic surface-active agent comprising a mixture of 75% of sodium tetradecenesulfonate and 25% of sodium hydroxytetradecanesulfonate, the components were uniformly mixed, and the obtained mixture was passed 2 times through a high-pressure homogenizer operating under pressure of 40 MPa. As a result, a uniform emulsion was obtained. The emulsion was then transferred to a separable flask equipped with a nitrogen-supply port and a stirrer. After 50 parts of an aqueous solution of 7% hydrochloric acid were added to the emulsion while the latter was stirred, the emulsion was retained for 24 hours at 25°C for emulsification polymerization. Upon completion of the emulsification polymerization, an aqueous solution of 5% sodium carbonate was added in an amount required for adjusting emulsion pH to 7. As a result, a polydimethylsiloxane emulsion was prepared. This emulsion had an average particle size of 0.50 μm, and the number-average molecular weight of polydimethylsiloxane was 146,000. This emulsion was designated as Sample B-6.

[Comparative Example 7]

499 parts of a α, ω-dihydroxypolydimethylsiloxane with a molecular weight of 3000 were added to a liquid mixture of 390 parts of ion-exchange water with 10 parts of dodecylbenzenesulfonic acid. The components were uniformly mixed, and the obtained mixture was passed 2 times through a high-pressure homogenizer operating under pressure of 40 MPa. As a result, a uniform emulsion was obtained. The emulsion was then transferred to a separable flask equipped with a nitrogen-supply port and a stirrer. After 50 parts of an aqueous solution of 7% hydrochloric acid were added to the emulsion while the latter was stirred, the emulsion was retained for 12 hours at 25°C, combined with 1 part of a slowly added methyltriethoxysilane, and held for another 12 hours at 25°C for emulsification polymerization. Upon completion of the emulsification polymerization, an aqueous solution of 5% sodium carbonate was added in an amount required for adjusting emulsion pH to 7. As a result, a polyorganosiloxane emulsion was prepared. This emulsion had an average particle size of 0.52 μm, and the number-average molecular weight of polydimethylsiloxane was 155,000. This emulsion was designated as Sample B-7.

[Practical Example 8]

Skin shampoo compositions A-10 and A-11 having components shown in Table 4 were prepared with the use of samples A-8 and A-9 produced in Practical Example 6 and 7. Stability of the aforementioned skin shampoo compositions was evaluated by the same method as in Practical Example 5. Furthermore, the touch sensation of skin washed with the use of the aforementioned shampoos was evaluated by a panel of 5 people in accordance with the method described below. The results are shown in Table 4.

Touch Sensation of Skin

After treating an arm for 30 sec. with the use of the skin shampoo, the treated portion was washed in a water flow. The remaining water was wiped off with a dry towel, and the touch sensation was evaluated after the treated skin was dried.

[Comparative Example 8]

Skin shampoo compositions B-8 and B-9 were prepared as in Practical Example 8 with the use of samples B-6 and B-7 produced in Comparative Example 6 and 7. The aforementioned skin shampoo compositions were evaluated with regard to stability and the touch sensation of skin. The results are shown in Table 4.

[Table 4]

| Skin shampoo composition No. | | | Practical Examples | | Comp. Examples | |
|---|---|---|---|---|---|---|
| . | | | A-10 | A-11 | B-8 | B-9 |
| Composition (%) | Lauroyl sarcosine sodium (30% aqueous solution) | | 15.0 | 15.0 | 15.0 | 15.0 |
| | Lauryl 2 sodium sulfosuccinate (27% aqueous solution) | | 15.0 | 15.0 | 15.0 | 15.0 |
| | Sample A-8 | | 10.0 | - | - | - |
| | Sample A-9 | | - | 10.0 | - | - |
| | Sample B-6 | | - | - | 10.0 | - |
| | Sample B-7 | | - | - | - | 10.0 |
| | Ion-exchange water | | 60.0 | 60.0 | 60.0 | 60.0 |
| Results | Stability | Directly after preparation | ◎ | ◎ | ◎ | ◎ |
| | | After 1 month at 50°C | ◎ | ◎ | ○ | ○ |
| | Evaluation of touch sensation | | Pleasant moist feeling on skin | Pleasant moist feeling on skin | Insufficient moist feeling, sensation of slightly dry skin | Insufficient moist feeling, sensation of slightly dry skin |

Commercial Applicability

Since the polyorganosiloxane emulsion of the invention is stable in storage and has good compounding stability with regard to various cosmetic materials, it is suitable for addition to various cosmetic materials as a silicone component. The polyorganosiloxane emulsion of the invention can be used as a cosmetic raw material for compounding with other components, but not as a cosmetic material itself.

Furthermore, since the cosmetic raw material of the invention has excellent cosmetic functions, such as affinity to skin and hair and imparting smoothness to skin and hair, it can be used as a cosmetic raw material for skin and hair.

## Claims

1. A polyorganosiloxane emulsion comprising: (A) polyorganosiloxane; (B) N-acylalkyltaurine and/or a salt thereof; and (C) water, said emulsion having an average particle diameter of 0.15 μm or more.

2. The polyorganosiloxane emulsion of Claim 1, wherein said component (A) is a dimethylpolysiloxane.

**3.** The polyorganosiloxane emulsion of Claim 1, wherein a number-average molecular weight of said component (A) is between 1,000 and 1,000,000.

**4.** The polyorganosiloxane emulsion of Claim 1, wherein said component (B) is a compound expressed by the following general formula (I):

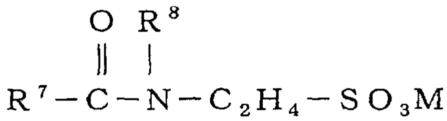

· · · (I) ,

where $R^7$ and $R^8$ are substituted or unsubstituted monovalent hydrocarbon groups, and M is a hydrogen atom, alkaline metal, ammonium, or a triethanolammonium).

**5.** The polyorganosiloxane emulsion of Claim 1, wherein said component (B) is a compound selected from the group consisting of sodium N-lauroylmethyltaurine, sodium N-myristoylmethyltaurine, sodium N-oleoylmethyltaurine, sodium N-stearoylmethyltaurine, sodium N-coconut oil fatty acid methyltaurate, potassium N-coconut oil fatty acid methyltaurate, magnesium N-coconut oil fatty acid methyltaurate, sodium N-palmytoylmethyltaurate, potassium N-stearoylmethyltaurate, potassium N-cetyloylmethyltaurate, and non-neutralized compounds of the above.

**6.** The polyorganosiloxane emulsion of Claim 1 that contains component (B) in an amount of 1 to 300 parts by weight and component (C) in an amount of 10 to 2000 parts by weight for each 100 parts by weight of component (A).

**7.** The polyorganosiloxane emulsion of Claim 1, which is obtained by emulsification polymerization.

**8.** The polyorganosiloxane emulsion of Claim 7, wherein the emulsification polymerization is carried out with the use of an acid catalyst.

**9.** A method of preparation of the polyorganosiloxane emulsion of Claim 1, **characterized by** subjecting a polyorganosiloxane (a) having a molecular weight lower than that of component (A), to emulsification polymerization in water in the presence of (B) N-acylalkyltaurine and/or a salt thereof.

**10.** The method of Claim 9, wherein after emulsification of said polyorganosiloxane (a) having a molecular weight lower than that of component (A) in water in the presence of (B) N-acylalkyltaurine and/or a salt thereof, emulsification polymerization is carried out with the addition of an acid catalyst.

**11.** The method of Claim 9, wherein said component (a) is a cyclic polyorganosiloxane or a mixture of a cyclic polyorganosiloxane with a linear-chain polydiorganosiloxane.

**12.** The method of Claim 9, wherein said component (a) is a polydiorganosiloxane having both molecular terminals capped with silanol groups.

**13.** The method of Claim 9, wherein a hydrolysable organosilane is added to said component (a) and then both said components are subjected to emulsification copolymerization.

**14.** A cosmetic raw material comprising the polyorganosiloxane emulsion according to any of Claims from 1 to 13.

**15.** The cosmetic raw material of Claim 14, wherein said cosmetic material is a hair cosmetic material.

**16.** The cosmetic raw material of Claim 14, wherein said cosmetic material is a skin cosmetic material.

## INTERNATIONAL SEARCH REPORT

International application No.
PCT/JP02/02346

A. CLASSIFICATION OF SUBJECT MATTER
Int.Cl[7] C08L83/04, A61K7/00, A61K7/06, C08G77/06, A61K7/075, A61K7/48

According to International Patent Classification (IPC) or to both national classification and IPC

B. FIELDS SEARCHED

Minimum documentation searched (classification system followed by classification symbols)
Int.Cl[7] C08G77/00-62, A61K7/00-50, C08L83/00-16

Documentation searched other than minimum documentation to the extent that such documents are included in the fields searched

| | | | |
|---|---|---|---|
| Jitsuyo Shinan Koho | 1926-1996 | Jitsuyo Shinan Toroku Koho | 1996-2002 |
| Kokai Jitsuyo Shinan Koho | 1971-2002 | Toroku Jitsuyo Shinan Koho | 1994-2002 |

Electronic data base consulted during the international search (name of data base and, where practicable, search terms used)
CAS ONLINE, WPI/L

C. DOCUMENTS CONSIDERED TO BE RELEVANT

| Category* | Citation of document, with indication, where appropriate, of the relevant passages | Relevant to claim No. |
|---|---|---|
| A | JP 3-17006 A (Gakken Co., Ltd.), 25 January, 1991 (25.01.91), Claims 1 to 4 (Family: none) | 1-16 |
| A | JP 4-227932 A (Dow Corning Corp.), 18 August, 1992 (18.08.92), Claim 1; Par. No. [0028] & EP 459500 A2 | 1-16 |
| A | JP 4-48925 A (Kanebo, Ltd.), 18 February, 1992 (18.02.92), Claim 1; page 3, lower right column, lines 4 to 6 (Family: none) | 1-16 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 June, 2002 (25.06.02) | 09 July, 2002 (09.07.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/02346

C (Continuation). DOCUMENTS CONSIDERED TO BE RELEVANT

| Category* | Citation of document, with indication, where appropriate, of the relevant passages | Relevant to claim No. |
|---|---|---|
| P,A | JP 2002-20490 A (GE Toshiba Silicones Co., Ltd.), 23 January, 2002 (23.01.02), Claims 1 to 5; Par. No. [0037] (Family: none) | 1-16 |
| P,A | EP 1151743 A2 (Kao Corp.), 07 November, 2001 (07.11.01), Claims 1 to 6; inventive examples 5, 8; comparative example 2 & JP 2002-12517 A & JP 2002-87931 A & US 2001/0053376 A1 | 1-16 |
| P,A | JP 2001-157835 A (Kao Corp.), 12 June, 2001 (12.06.01), Claims 1 to 3; comparative example 1 (Family: none) | 1-16 |

Form PCT/ISA/210 (continuation of second sheet) (July 1998)